(19) **Europäisches Patentamt / European Patent Office / Office européen des brevets**

(11) **EP 3 929 933 A1**

(12) # EUROPEAN PATENT APPLICATION
published in accordance with Art. 153(4) EPC

(43) Date of publication:
**29.12.2021 Bulletin 2021/52**

(51) Int Cl.:
***G16H 30/20*** (2018.01)

(21) Application number: **19915864.3**

(22) Date of filing: **16.08.2019**

(86) International application number:
**PCT/CN2019/101158**

(87) International publication number:
**WO 2020/168696 (27.08.2020 Gazette 2020/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.02.2019 CN 201910132134**

(71) Applicant: **VR Doctor Medical Technology (Shenzhen) Co., Ltd.**
**Shenzhen, Guangdong, 518035 (CN)**

(72) Inventors:
• **LEE, Stewart Ping**
  **Shenzhen, Guangdong 518035 (CN)**
• **LEE, David Wei**
  **Shenzhen, Guangdong 518035 (CN)**

(74) Representative: **Dargiewicz, Joanna**
**JD&P Patent Attorneys**
**Joanna Dargiewicz & Partners**
**Ul. Mysliborska 93A/50**
**03-185 Warszawa (PL)**

(54) **VRDS 4D MEDICAL IMAGE-BASED ARTERY AND VEIN AI PROCESSING METHOD AND PRODUCT**

(57) A method and a product for AI processing of artery and vein based on VRDS 4D medical images, the method is applied to a medical imaging apparatus, and the method includes: the medical imaging apparatus first determines a bitmap (BMP) data source according to a plurality of scanned images of a target site of a target user, second generates target medical image data according to the BMP data source, and finally performs 4D medical imaging according to the target medical image data, wherein the target medical image data includes at least a data set of a blood vessel in the target site, and a data set of a vein is a transfer function result of a cubic space of a surface of the vein and a tissue structure inside the vein, and an intersection position of the artery and a vein presents an overall separation image effect.

Determining, by medical imaging apparatus, a bitmap (BMP) data source according to a plurality of scanned images of a target site of a target user — S201

Generating , by the medical imaging apparatus, target medical image data according to the BMP data source — S202

Performing , by the medical imaging apparatus, a 4D medical imaging according to the target medical image data — S203

Fig. 2

EP 3 929 933 A1

## Description

## TECHNICAL FIELD

This application relates to the field of medical imaging apparatus, and in particular, to a method and a product for AI processing of artery and vein based on VRDS 4D medical images.

## BACKGROUND

At present, doctors use technologies such as Computed Tomography (CT), Magnetic Resonance Imaging (MRI), Diffusion Tensor Imaging (DTI), Positron Emission Computed Tomography (PET) to acquire information such as the shape, position and topology of the internal tissues of the human body. However, the image of blood vessels formed by two-dimensional slice data is ambiguous. With rapid development of medical imaging technology, people put forward new demands for medical imaging of blood vessels.

## SUMMARY

Embodiments of this application provide a method and a product for AI processing of artery and vein based on VRDS 4D medical images, in order to improve the accuracy and efficiency of the medical imaging apparatus in imaging the artery and vein.

In a first aspect, embodiments of this application provide a method for AI processing of artery and vein based on VRDS 4D medical images, which is applied to medical imaging apparatus. The method includes:

determining a bitmap (BMP) data source according to a plurality of scanned images of a target site of a target user;

generating target medical image data according to the BMP data source, wherein the target medical image data includes at least a data set of a blood vessel of the target site, the data set of the blood vessel includes a data set of an artery and a data set of a vein; and first data in the data set of the artery and second data in the data set of the vein are independent of each other; the first data is data associated with an intersection position of the artery and the vein, the second data is data associated with the intersection position, the data set of artery is a transfer function result of a cubic space between a surface of the artery and a tissue structure inside the artery, and the data set of the vein is a transfer function result of a cubic space between a surface of the vein and a tissue structure inside the vein;

performing a 4D medical imaging according to the target medical image data, wherein the intersection position of the artery and the vein presents an overall separation image effect.

In a second aspect, embodiments of this application provide an apparatus for AI processing of artery and vein based on VRDS 4D medical images, which is applied to a medical imaging apparatus. The apparatus for AI processing of artery and vein based on VRDS 4D medical image includes a processing unit and a communication unit, wherein,

the processing unit is configured to: determine a BMP data source according to a plurality of scanned images of a target site of a target user; generate target medical image data according to the BMP data source, wherein the target medical image data includes at least a data set of a blood vessel of the target site, the data set of the blood vessel includes a data set of an artery and a data set of a vein; and first data in the data set of the artery and second data in the data set of the vein are independent of each other, the first data is data associated with the intersection position of the artery and the vein, the second data is data associated with the intersection position, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel; and perform 4D medical imaging according to the target medical image data through the communication unit, wherein the intersection position of the artery and the vein presents an overall separation image effect.

In a third aspect, embodiments of this application provide an medical imaging apparatus, including a processor, a memory, a communication interface, and one or more programs, wherein the one or more programs are stored in the memory and configured to be executed by the processor, and the programs include instructions for executing the steps in any method of the first aspect of the embodiment of this application.

In a fourth aspect, embodiments of this application provide a computer readable storage medium, wherein the computer readable storage medium stores a computer program for electronic data exchange, wherein the computer program causes a computer to execute some or all of the steps described in any methods of the first aspect of the embodiment of this application.

In a fifth aspect, embodiments of this application provide a computer program product, wherein the computer program product includes a non-transitory computer-readable storage medium in which a computer program is stored, the computer program is operable to cause the computer to execute some or all of the steps described in any methods of the first aspect of the embodiment of this application. The computer program product can be a software installation package.

As can be seen that in the embodiment of this application, first, the medical imaging apparatus determines a BMP data source according to a plurality of scanned images of a target site of a target user; second, generates target medical image data according to the BMP data source, and finally performs 4D medical imaging according to the target medical image data, wherein the target medical image data includes at least a data set of the blood vessel of the target site, the data set of the blood vessel includes a data set of an artery and a data set of a vein, and first data in the data set of the artery and second data in the data set of the vein are independent of each other, the first data is data associated with the intersection position of the artery and the vein, the second data is data associated with the intersection position, the data set of the artery is a transfer function result of a cubic space between a surface of the artery and a tissue structure inside the artery, and the data set of the vein is a transfer function result of a cubic space between a surface of the vein and a tissue structure inside the vein, and the intersection position of the artery and the vein presents an overall separation image effect, thus, the medical imaging apparatus of this application can perform 4D medical image displaying based on raw scanned images, which is facilitated to improve the accuracy and efficiency of artery and vein imaging.

## BRIEF DESCRIPTION OF THE DRAWINGS

In order to describe the technical solutions in the embodiments of this application or in the prior art more clearly, the following briefly describes the accompanying drawings required for describing the embodiments or the prior art, apparently, the accompanying drawings in the following description only show some embodiments of this application, and the ordinary skill person in the art may still derive other drawings from these accompanying drawings without creative efforts.

Fig. 1 is a schematic structural diagram of an intelligent analyzing and processing system based on VRDS AI 4D medical images provided by an embodiment of this application;

Fig. 2 is a schematic flowchart of a method for AI processing of artery and vein based on VRDS 4D medical images provided by an embodiment of this application;

Fig. 3 is a schematic structural diagram of an medical imaging apparatus provided by an embodiment of this application;

Fig. 4 is a block diagram of functional units composition of an apparatus for AI processing of artery and vein based on VRDS AI 4D medical images provided by an embodiment of this application.

## DETAILED DESCRIPTION OF PREFERRED EMBODIMENTS

In order to make the skilled person in the art better understand the solution of this application, the following clearly and completely describes the technical solutions in the embodiments of this application with reference to the accompanying drawings in the embodiments of this application, apparently, the described embodiments are only some but not all of the embodiments of this application. All other embodiments obtained by the ordinary skill person in the art based on the embodiments of this application without creative efforts shall fall within the protection scope of this application.

The terms "first", "second", etc. in the specification and claims of this application and the above drawings are used to distinguish different objects, but not to describe a specific order. Furthermore, that term "comprising" and "have" and any variations thereof are intended to cover non-exclusive inclusion. For example, a process, method, system, product or device including a series of steps or units is not limited to the listed steps or units, but optionally further includes steps or units not listed, or optionally further includes other steps or units inherent to these processes, methods, products or devices.

Reference to an "embodiment" herein means that a particular feature, structure or characteristic described in connection with an embodiment may be included in at least one embodiment of this application. The appearances of the phrase in various places in the specification are not necessarily all referring to the same embodiment, nor are separate or alternative embodiments mutually exclusive of other embodiments. It is explicitly and implicitly to be understood by the skilled person in the art that the described embodiment may be combined with other embodiments.

The medical imaging apparatuses related to the embodiments of this application refer to various instruments that use various different media as information carriers to reproduce the internal structure of the human body as images, and their image information has a corresponding relationship in spatial and temporal distribution with the actual structure of the human body. "DICOM data" refers to the raw image file data collected by medical device and reflecting the internal structure features of human body, and may include information such as Computed Tomography (CT), Nuclear Magnetic Resonance (MRI), Diffusion Tensor Imaging (DTI), Positron Emission Computed Tomography (PET-CT). "Image Source" refers to Texture 2D/3D image volume data generated by parsing the raw DICOM data. "VRDS" refers to Virtual Reality Doctor system.

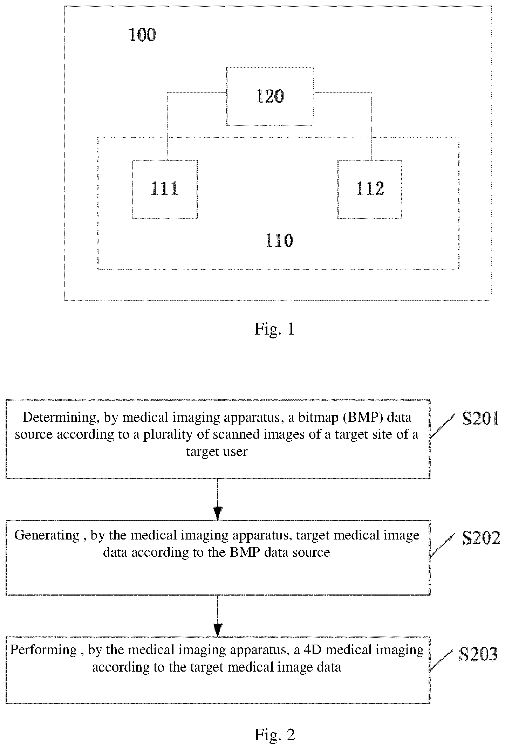

Referring to Fig. 1, Fig. 1 is a schematic structural diagram of an intelligent analyzing and processing system 100 based on VRDS AI 4D medical images provided by an embodiment of this application, the system 100 includes a medical imaging apparatus 110 and a network database 120, wherein the medical imaging apparatus 110 may include a local medical imaging apparatus 111 and/or a terminal medical imaging apparatus 112, the local medical imaging apparatus 111 or the terminal medical imaging apparatus 112 is configured to perform drawing on a four-dimensional separation volume of artery and vein of human body based on the raw DICOM data and the method for analyzing and processing of arteries and veins based on VRDS AI 4D medical image presented in the embodiment of this application, so as to achieve the four-dimensional stereo effect (the 4-dimensional medical image specifically refers to the medical image including the internal spatial structure features and the external spatial structure features of the displayed tissue, the internal spatial structure features refer to slice data inside the tissues are not lost, that is, medical imaging apparatuses can present the internal constructions of tissues such as target organs and blood vessels, and the external spatial structure characteristics refer to the environmental features between tissues, including spatial position characteristics (including intersection, spacing and fusion) between tissues, such as edge structure characteristics of the intersection position between kidney and artery, etc.), compared with the terminal medical imaging apparatus 112, the local medical imaging apparatus 111 can further be configured to edit the image source data to form the transfer function results of the four-dimensional human body image, which can include the transfer function results of the surface of the internal organs of the human body and the tissue structure inside the internal organs of the human body, as well as the transfer function results of the cube space, such as the number, coordinates, colors, transparency and other information of the cube editing boxes and arc editing arrays required by the transfer function. The network database 120 can be, for example, a cloud server, etc., the network database 120 is configured to store the image source generated by parsing the raw DICOM data and the transfer function result of the four-dimensional human body image edited by the local medical imaging apparatus 111, the image source can come from a plurality of local medical imaging apparatuses 111 to achieve the interactive diagnosis of a plurality of doctors.

When a user performs a specific image displaying through the above medical imaging apparatus 110, the user can choose a display or a Head mounted Displays Set (HMDS) of virtual reality VR to display in combination with operation actions, which refer to the operation control of the four-dimensional human body image by the user through external intake device of the medical imaging apparatus, such as a mouse and a keyboard, so as to achieve human-computer interaction. The operation actions include at least one of the following: (1) changing the color and/or transparency of a specific organ/tissue, (2) positioning and scaling the view, (3) rotating the view to achieve multi-view 360-degree observation of the four-dimensional human image, (4) "entering" inside the organ of the human body to observe the internal construction, and the shearing effect rendering in real time, and (5) moving the view up and down.

The following describes a method for AI processing of artery and vein based on VRDS 4D medical images in detail.

Referring to Fig. 2, Fig. 2 is a schematic flowchart of a method for AI processing of artery and vein based on VRDS 4D medical images provided by an embodiment of this application, which is applied to medical imaging apparatus as described in Fig. 1; as shown in the figure, the method for AI processing of artery and vein based on VRDS 4D medical images includes:

S201, determining, by a medical imaging apparatus, a BMP data source according to a plurality of scanned images of a target site of a target user; wherein the target site is, for example, a kidney. And the scanned image includes any one of the following: a CT image, an MRI image, a DTI image and a PET-CT image.

In one possible example, the medical imaging apparatus determines the BMP data source according to a plurality of scanned images associated with the target organ of the target user, including: the medical imaging apparatus acquires a plurality of scanned images collected by a medical device and reflecting internal structure features of human body of the target user; screens at least one scanned image including the target site from the plurality of scanned images; and takes the at least one scanned image as Digital Imaging and Communications in Medicine (DICOM) data of the target user; parses the DICOM data to generate an image source of the target user, wherein the image source includes Texture 2D/3D image volume data; executes a second preset process for the image source to obtain the BMP data source, wherein the second preset process includes at least one of the following operations: VRDS limited contrast adaptive histogram equalization, mixed partial differential denoising and VRDS AI elastic deformation processing.

It can be seen that in this example, the medical imaging apparatus will screen the scanned images to obtain the DICOM data, and then parse the DICOM data to obtain Texture 2D/3D image volume data, finally, through at least one of equalization, denoising and elastic deformation, the BMP data source meeting the requirements of medical images is obtained.

In this possible example, the VRDS limited contrast adaptive histogram equalization includes: regional noise ratio limiting and global contrast limiting; the local histogram of the image source is divided into a plurality of partitions, for each partition, the slope of the transform function is determined according to the slope of the cumulative histogram of the neighborhood of the partition, and the contrast amplification degree around the pixel value of the partition is determined according to the slope of the transform function, then, according to the contrast amplification degree, the limit clipping process is carried out to generate the distribution of effective histograms and the value of effective available neighborhood size, and these clipped partial histograms are uniformly distributed to other areas of the histogram.

The mixed partial differential denoising includes: different from Gaussian low-pass filtering (which weakens the high-frequency components of the image indiscriminately and blurs the edges of the image while denoising), the isophotes (including edges) formed by objects in the natural image should be smooth and unhindered curves, that is, the absolute value of curvature of these isophotes should be small enough, and when the image is polluted by noise, the local gray value of the image will fluctuate randomly, and it leads to the irregular oscillation of the isophote and forms the isophote with large local curvature, and according to this principle, a mixed partial differential denoising model is designed, which can protect the image edge and avoid the step effect in the smoothing process by VRDS AI curvature driving and VRDS AI high-order hybrid denoising.

The VRDS AI elastic deformation processing includes: superimposing positive and negative random distances on the original lattice to form a difference position matrix, and then forming a new lattice at the gray level of each difference position, so as to achieve the internal distortion of the image, and further performing rotation, distortion and translation operations etc. on the image.

It can be seen that in this example, the medical imaging apparatus obtains the BMP data source by processing the raw scanned image data, which increases the information amount of the raw data and increases the depth dimension information, and finally obtains the data meeting the display requirements of the 4D medical image.

S202, generating, by the medical imaging apparatus, target medical image data according to the BMP data source, wherein the target medical image data includes at least a data set of a blood vessel of the target site, the data set of the blood vessel includes a data set of an artery and a data set of a vein; and first data in the data set of the artery and second data in the data set of the vein are independent of each other, the first data is data associated with the intersection position of the artery and the vein, the second data is data associated with the intersection position, the data set of artery is a transfer function result of a cubic space between a surface of the artery and a tissue structure inside the artery, and the data set of the vein is a transfer function result of a cubic space between a surface of the vein and a tissue structure inside the vein;

In one possible example, the medical imaging apparatus generates target medical image data according to the BMP data source, including: the medical imaging apparatus introduces the BMP data source into a preset VRDS medical network model to obtain first medical image data, wherein the first medical image data includes the data set of the blood vessel of the target site; the data set of the blood vessel includes fusion data of the intersection position, data of the artery excluding the intersection position and data of the vein excluding the intersection position; and the data set of the blood vessel is a transfer function result of a cubic space between a surface of the blood vessel and a tissue structure inside the blood vessel; introduces the first medical image data into a preset cross blood vessel network model and performs spatial segmentation processing on the fusion data at the intersection positions by the cross blood vessel network model to obtain the first data and the second data; synthesizes the first data, the second data, the data of the artery excluding the intersection position and the data of the vein excluding the intersection position to obtain the target medical image data.

It can be seen that in this example, the medical imaging apparatus introduces the BMP data source into a preset VRDS medical network model to obtain first medical image data, and then introduces the first medical image data into a preset cross blood vessel network model and performs spatial segmentation processing on the fusion data at the intersection positions by the cross blood vessel network model to obtain the first data and the second data; synthesizes the first data, the second data, the data of the artery excluding the intersection position and the data of the vein excluding the intersection position to obtain the target medical image data, which is facilitated to acquire the target medical image data more accurately and comprehensively and improve the accuracy of the target medical image data.

In this possible example, the medical imaging apparatus synthesizes the first data, the second data, the data of the artery excluding the intersection position and the data of the vein excluding the intersection position to obtain the target medical image data, including: the medical imaging apparatus synthesizes the first data, the second data, the data of the artery excluding the intersection position and the data of the vein excluding the intersection position to obtain second medical image data; executes a first preset processing on the second medical image data to obtain the target medical image data, wherein the first preset processing includes at least one of the following operations: 2D boundary optimization processing, 3D boundary optimization processing and data enhancement processing, and the target medical image data includes the data set of the target organ, the data set of the artery and the data set of the vein;

Wherein the 2D boundary optimization processing includes: acquiring low-resolution information and high-resolution information by sampling multiple times, wherein the low-resolution information can provide context semantic information of a segmentation target throughout the image, that is, features reflecting the relationship between the target and the environment, these features are used to judge the class of objects, and the high-resolution information is used to provide more fine features, such as gradients, for segmentation targets.

Wherein, the 3D boundary optimization processing includes: 3D convolution, 3D max-pooling and 3D upward convolution layer. The size of input data is a1, a2 and a3, the number of channels is c, and the size of the filter is f, that is, the dimension of the filter is f*f*f*c, and the number of filters is n, so the final output of 3 dimensional convolution is:

$$(a1-f+1)*(a2-f+1)*(a3-f+1)*n,$$

Which has an analysis path and a synthesis path. In the analysis path, each layer includes two convolution kernels of 3*3*3, each of which follows an activation function (Relu), and then there is a max-pooling of 2*2*2 on each dimension to merge two step lengths. In the synthesis path, each layer is composed of 2*2*2 upward convolution, and the step length on each dimension is 2, next, two 3*3*3 convolutions, and then Relu. Shortcut connections from equal resolution layers in the analysis path then provide the basic high-resolution features of the synthetic path. In the last layer, 1*1*1 convolution reduces the number of output channels.

Wherein, the data enhancement processing includes any one of the following: data enhancement based on arbitrary angle rotation, data enhancement based on histogram equalization, data enhancement based on white balance, data enhancement based on mirror operation, data enhancement based on random shearing and data enhancement based on simulating different illumination changes.

It can be seen that in this example, the medical imaging apparatus synthesizes the first data, the second data, the data of the artery excluding the intersection position and the data of the vein excluding the intersection position to obtain second medical image data; executes a first preset processing on the second medical image data to obtain target medical image data; performs further optimization processing on the obtained medical image data, which is facilitated to improve the accuracy and efficiency of the target medical image data.

In this possible example, the medical imaging apparatus introduces the first medical image data into a preset cross blood vessel network model and performs spatial segmentation processing on the fusion data at the intersection position by the cross blood vessel network model to obtain the first data and the second data, including: the medical imaging apparatus partitions the intersection position according to an intersection complexity to obtain a plurality of cross partitions; screens fusion data of each cross partition to reduce the data volume of each partition; introduces the screened data of each partition into the cross blood vessel network model to obtain a first partition data and a second partition data of each partition; synthesizes a plurality of pieces of first partition data of the plurality of cross partitions to obtain the first data, and synthesizes a plurality of pieces of second partition data of the plurality of cross partitions to obtain the second data.

In specific implementation, the intersection complexity of the intersection position can be calculated according to the curvature range of the intersection area, and the larger the curvature range, the closer and more complex the contact between artery and vein, and the higher the corresponding intersection complexity. Partitioning at the intersection position can be specifically partitioned according to the curvature radius, for example, the area within the radius of curvature a1 to a2 is a first intersection partition, and the area within the radius of curvature a2 to a3 is a second intersection partition. The screening strategy of the data of each intersection partition can be executed by convolution operation or sparse processing, aiming at reducing the amount of data and improving the computational efficiency on the basis of ensuring the display effect.

Wherein, the cross blood vessel network model achieves the data separation of arteries and veins by the following operations: (1) extracting the fusion data of the intersection position; (2) for each fusion data, separating the fusion data based on a preset data separation algorithm to obtain artery boundary point data and vein boundary point data that are independent of each other; (3) integrating multiple artery boundary point data obtained after processing into the first data and integrating multiple vein boundary point data obtained after processing into the second data.

It can be seen that in this example, the medical imaging apparatus can perform partitioning and sparse processing on the data of the intersection position of artery and vein, which ensures the display effect, improves the calculation efficiency and the real-time performance.

S203, performing, by the medical imaging apparatus, a 4D medical imaging according to the target medical image data, wherein the intersection position of the artery and the vein presents an overall separation image effect;

wherein, the 4D medical imaging refers to presenting the 4-dimensional medical image.

In one possible example, the medical imaging apparatus preforms the 4D medical imaging according to the target medical image data, including: the medical imaging apparatus screens enhanced data with a quality score greater than a preset score from the target medical image data as VRDS 4D imaging data; and performs 4D medical imaging according to the VRDS 4D imaging data.

Wherein, the quality score can be comprehensively evaluated from the following dimensions: average gradient, information entropy, visual information fidelity, peak signal-to-noise ratio (PSNR), structural similarity index measurement (SSIM), mean square error (MSE), etc., specific reference can be made to common image quality score algorithms in the image field, which is not described details for brevity.

It can be seen that, in this example, the medical imaging apparatus further performs data screening through the quality score, thus improving the imaging effect.

As can be seen that in the embodiment of this application, first, the medical imaging apparatus determines a BMP data source according to a plurality of scanned images of a target site of a target user; second, generates target medical image data according to the BMP data source, and finally performs 4D medical imaging according to the target medical image data, wherein the target medical image data includes at least a data set of the blood vessel of the target site; the data set of the blood vessel includes a data set of an artery and a data set of a vein; and first data in the data set of the artery and second data in the data set of the vein are independent of each other, the first data is data associated with the intersection position of the artery and the vein, the second data is data associated with the intersection position, the data set of the artery is a transfer function result of a cubic space between a surface of the artery and a tissue structure inside the artery, and the data set of the vein is a transfer function result of a cubic space between a surface of the vein and a tissue structure inside the vein, and the intersection position of the artery and the vein presents an overall separation image effect, thus, the medical imaging apparatus of this application can perform 4D medical image displaying based on the raw scanned images, which is beneficial to improve the accuracy and efficiency of artery and vein imaging.

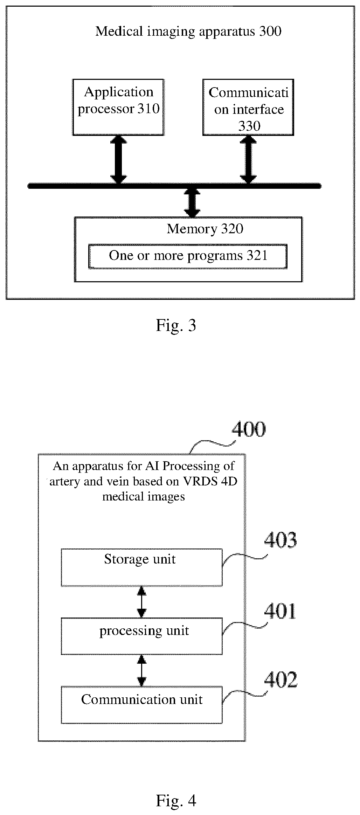

Consistent with the embodiments shown in Fig. 2, referring to Fig. 3, Fig. 3 is a schematic structural diagram of a medical imaging apparatus 300 provided by an embodiment of this application, as shown in the figure, the medical imaging apparatus 300 includes a processor 310, a memory 320, a communication interface 330 and one or more programs 321, wherein the one or more programs 321 are stored in the memory 320 and configured to be executed by the processor 310, and the one or more programs 321 include instructions for executing the following steps:

determining a BMP data source according to a plurality of scanned images of a target site of a target user;

generating target medical image data according to the BMP data source, wherein the target medical image data includes at least a data set of a blood vessel of the target site, the data set of the blood vessel includes a data set of an artery and a data set of a vein; and first data in the data set of the artery and second data in the data set of the vein are independent of each other, the first data is data associated with the intersection position of the artery and the vein, the second data is data associated with the intersection position, the data set of artery is a transfer function result of a cubic space between a surface of the artery and a tissue structure inside the artery, and the data set of the vein is a transfer function result of a cubic space between a surface of the vein and a tissue structure inside the vein;

performing a 4D medical imaging according to the target medical image data, wherein the intersection position of the artery and the vein presents an overall separation image effect.

As can be seen that in the embodiment of this application, first, the medical imaging apparatus determines a BMP data source according to a plurality of scanned images of a target site of a target user; second, generates target medical image data according to the BMP data source, and finally performs 4D medical imaging according to the target medical image data, wherein the target medical image data includes at least a data set of a blood vessel of the target site, the data set of the blood vessel includes a data set of an artery and a data set of a vein; and first data in the data set of the artery and second data in the data set of the vein are independent of each other, the first data is data associated with the intersection position of the artery and the vein, the second data is data associated with the intersection position, the data set of the artery is a transfer function result of a cubic space between a surface of the artery and a tissue structure inside the artery, and the data set of the vein is a transfer function result of a cubic space between a surface of the vein and a tissue structure inside the vein, and the intersection position of the artery and the vein presents an overall separation image effect, thus, the medical imaging apparatus of this application can perform 4D medical image displaying based on the raw scanned images, which is facilitated to improve the accuracy and efficiency of artery and vein imaging.

In one possible example, in the aspect of generating of target medical image data according to the BMP data source, the program further includes instructions configured to perform the following operation: introducing the BMP data source into a preset VRDS medical network model to obtain a first medical image data, wherein the first medical image data includes the data set of the blood vessel of the target site, the data set of the blood vessel includes fusion data of the intersection position, data of the artery excluding the intersection position and data of the vein excluding the intersection position; and the data set of the blood vessel is a transfer function result of a cubic space between a surface of the blood vessel and a tissue structure inside the blood vessel; introducing the first medical image data into a preset cross blood vessel network model and performing spatial segmentation processing on the fusion data at the intersection positions by the cross blood vessel network model to obtain the first data and the second data; synthesizing the first data, the second data, the data of the artery excluding the intersection position and the data of the vein excluding the intersection position to obtain the target medical image data.

In one possible example, in the aspect of the synthesizing of the first data, the second data, the data of the artery excluding the intersection position and the data of the vein excluding the intersection position to obtain the target medical image data, the instructions in the program are specifically configured to perform the following operation: synthesizing the first data, the second data, the data of the artery excluding the intersection position and the data of the vein excluding the intersection position to obtain a second medical image data; executing a first preset processing on the second medical image data to obtain target medical image data, wherein the first preset processing includes at least one of the following operations: 2D boundary optimization processing, 3D boundary optimization processing and data enhancement processing, and the target medical image data includes the data set of the target organ, the data set of the artery and the data set of the vein.

In one possible example, in the aspect of introducing of the first medical image data into a preset cross blood vessel network model and performs spatial segmentation processing on the fusion data at the intersection position by the cross blood vessel network model to obtain the first data and the second data, the program further includes instructions configured to perform the following operation: partitioning the intersection position according to the intersection complexity to obtain a plurality of cross partitions; screening fusion data of each cross partition to reduce the data volume of each cross partition; introducing the screened data of each cross partition into the cross blood vessel network model to obtain a first partition data and a second partition data of each cross partition; synthesizing a plurality of pieces of first partition data of the plurality of cross partitions to obtain the first data, and synthesizing a plurality of pieces of second partition data of the plurality of cross partitions to obtain the second data.

In one possible example, in the aspect of performing of the 4D medical imaging according to the target medical image data, the program further includes instructions configured to perform the following operation: screening enhanced data with a quality score greater than a preset score from the target medical image data as VRDS 4D imaging data; performing 4D medical imaging according to the VRDS 4D imaging data.

In one possible example, in the aspect of determining of the BMP data source according to a plurality of scanned images associated with the target organ of the target user, the program further includes instructions configured to perform the following operation: acquiring a plurality of scanned images collected by a medical device and reflecting internal structure features of human body of the target user; screening at least one scanned image including the target site from the plurality of scanned images, and taking the at least one scanned image as Digital Imaging and Communications in Medicine (DICOM) data of the target user; parsing the DICOM data to generate an image source of the target user, wherein the image source includes a Texture 2D/3D image volume data; executing a second preset process for the image source to obtain the BMP data source, wherein the second preset process includes at least one of the following operations: VRDS limited contrast adaptive histogram equalization, mixed partial differential denoising and VRDS AI elastic deformation processing.

In one possible example, the VRDS limited contrast adaptive histogram equalization instructions are specifically configured to perform the following operation: regional noise ratio limiting and global contrast limiting; dividing the local histogram of the image source into a plurality of partitions, determining a slope of a transform function for each partition according to a slope of a cumulative histogram of a neighborhood of the partition, determining a contrast amplification degree around a pixel value of the partition according to the slope of the transform function, then performing a limit clipping process according to the contrast amplification degree to generate the distribution of effective histograms and a value of a size of an effective available neighborhood, and uniformly distributing these clipped partial histograms to other areas of the histogram; the mixed partial differential denoising instructions are specifically configured to perform the following operation: enabling the curvature of the image edge to be smaller than the preset curvature through VRDS AI curvature driving and VRDS AI high-order mixed denoising, thereby achieving a mixed partial differential denoising model capable of protecting an image edge and avoiding a step effect occurred during a smoothing process; the VRDS AI elastic deformation processing are specifically configured to perform the following operation: superimposing positive and negative random distances on an image lattice to form a difference position matrix, and then forming a new lattice at a gray level of each difference position, so as to achieve the internal distortion of an image, and then performing rotation, distortion and translation operations on the image.

The above mainly introduces the solution of the embodiment of this application from the perspective of the execution process on the method side. It can be understood that in order to achieve the above functions, the medical imaging apparatus includes corresponding hardware structures and/or software modules for performing various functions. It should be easy for the skilled person in the art aware that, in combination with the units and algorithmic steps of the examples described in the embodiments provided herein, this application can be implemented in the form of hardware or a combination of hardware and computer software. Whether the functions are performed by hardware or computer software driving hardware depends on particular applications and design constraint conditions of the technical solutions. A person skilled in the art may use different methods to implement the described functions for each particular application, but it should not be considered that the implementation goes beyond the scope of this application.

The embodiment of this application can divide the medical imaging apparatus into functional units according to the above method example, for example, individual functional unit can be divided corresponding to individual function, or two or more functions can be integrated into one processing unit. The integrated units can be implemented in the form of hardware, and can also be implemented in the form of a software functional unit. It should be noted that the division of units in the embodiment of this application is schematic, which is only a logical function division, and there may be another division mode in actual implementation.

Fig. 4 is a block diagram of functional units composition of an apparatus 400 for AI processing of artery and vein based on VRDS AI 4D medical images involved in the embodiment of this application. The apparatus 400 for AI processing of artery and vein based on VRDS AI 4D medical images is applied to a medical imaging apparatus, the apparatus 400 for AI processing of artery and vein based on VRDS AI 4D medical image includes a processing unit 401 and a communication unit 402, wherein,

the processing unit 401 is configured to: determine a BMP data source according to a plurality of scanned images of a target site of a target user; generate target medical image data according to the BMP data source, wherein the target medical image data includes at least a data set of a blood vessel of the target site, the data set of the blood vessel includes a data set of an artery and a data set of a vein; and first data in the data set of the artery and second data in the data set of the vein are independent of each other, the first data is data associated with the intersection position of the artery and the vein, the second data is data associated with the intersection position, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel; and perform 4D medical imaging according to the target medical image data through the communication unit 402, wherein the intersection position of the artery and the vein presents an overall separation image effect.

Wherein, the apparatus 400 further includes a storage unit 403, wherein the processing unit 401 can be a processor, the communication unit 402 can be a communication interface, and the storage unit 403 can be a memory.

As can be seen that in the embodiment of this application, first, the medical imaging apparatus determines a BMP data source according to a plurality of scanned images of a target site of a target user; second, generates a target medical image data according to the BMP data source, and finally performs 4D medical imaging according to the target medical image data, wherein the target medical image data includes at least a data set of a blood vessel of the target site, the data set of the blood vessel includes a data set of an artery and a data set of a vein; and first data in the data set of the artery and second data in the data set of the vein are independent of each other, the first data is data associated with the intersection position of the artery and the vein, the second data is data associated with the intersection position, the data set of the artery is a transfer function result of a cubic space between a surface of the artery and a tissue structure inside the artery, and the data set of the vein is a transfer function result of a cubic space between a surface of the vein and a tissue structure inside the vein, and the intersection position of the artery and the vein presents an overall separation image effect, thus, the medical imaging apparatus of this application can perform 4D medical image displaying based on the raw scanned images, which is facilitated to improve the accuracy and efficiency of artery and vein imaging.

In one possible example, the generating of target medical image data according to the BMP data source, the processing unit 401 is specifically configured to: introduce the BMP data source into a preset VRDS medical network model to obtain a first medical image data, wherein the first medical image data includes the data set of the blood vessel of the target site, the data set of the blood vessel includes fusion data of the intersection position, data of the artery excluding the intersection position and data of the vein excluding the intersection position; and the data set of the blood vessel is a transfer function result of a cubic space between a surface of the blood vessel and a tissue structure inside the blood vessel; introduce the first medical image data into a preset cross blood vessel network model and perform spatial segmentation processing on the fusion data at the intersection positions by the cross blood vessel network model to obtain the first data and the second data; synthesize the first data, the second data, the data of the artery excluding the intersection position and the data of the vein excluding the intersection position to obtain the target medical image data.

In one possible example, in the aspect of the synthesizing of the first data, the second data, the data of the artery excluding the intersection position and the data of the vein excluding the intersection position to obtain the target medical image data, the processing unit 401 is specifically configured to: synthesize the first data, the second data, the data of the artery excluding the intersection position and the data of the vein excluding the intersection position to obtain second medical image data; execute a first preset processing on the second medical image data to obtain the target medical image data, wherein the first preset processing includes at least one of the following operations: 2D boundary optimization processing, 3D boundary optimization processing and data enhancement processing, and the target medical image data includes the data set of the target organ, the data set of the artery and the data set of the vein.

In one possible example, the introducing of the first medical image data into a preset cross blood vessel network model and performs spatial segmentation processing on the fusion data at the intersection position by the cross blood vessel network model to obtain the first data and the second data, the processing unit 401 is specifically configured to: partition the intersection position according to the intersection complexity to obtain a plurality of cross partitions; screen fusion data of each cross partition to reduce the data volume of each cross partition; introduce the screened data of each cross partition into the cross blood vessel network model to obtain a first partition data and a second partition data of each cross partition; synthesize a plurality of pieces of first partition data of the plurality of cross partitions to obtain the first data, and synthesize a plurality of pieces of second partition data of the plurality of cross partitions to obtain the second data.

In one possible example, the performing of the 4D medical imaging according to the target medical image data, the processing unit 401 is specifically configured to: screen enhanced data with a quality score greater than a preset score from the target medical image data as VRDS 4D imaging data; perform 4D medical imaging according to the VRDS 4D imaging data.

In one possible example, the determining of the BMP data source according to a plurality of scanned images associated with the target organ of the target user, the processing unit 401 is specifically configured to: acquire a plurality of scanned images collected by a medical device and reflecting internal structure features of human body of the target user; screen at least one scanned image including the target site from the plurality of scanned images, and take the at least one scanned image as Digital Imaging and Communications in Medicine (DICOM) data of the target user; parse the DICOM data to generate a image source of the target user, wherein the image source includes Texture 2D/3D image volume data; execute a second preset process for the image source to obtain the BMP data source, wherein the second preset process includes at least one of the following operations: VRDS limited contrast adaptive histogram equalization, mixed partial differential denoising and VRDS AI elastic deformation processing.

In one possible example, in the VRDS limited contrast adaptive histogram equalization, the processing unit 401 is specifically configured to: limit a regional noise ratio and global contrast; divide the local histogram of the image source into a plurality of partitions; determine a slope of a transform function for each partition according to a slope of a cumulative histogram of a neighborhood of the partition; determine a contrast amplification degree around a pixel value of the partition according to the slope of the transform function; then perform a limit clipping process according to the contrast amplification degree to generate the distribution of effective histograms and a value of a size of an effective available neighborhood; and uniformly distribute these clipped partial histograms to other areas of the histogram; in the mixed partial differential denoising, the processing unit 401 is specifically configured to: enable the curvature of the image edge to be smaller than the preset curvature through VRDS AI curvature driving and VRDS AI high-order mixed denoising, thereby achieve a mixed partial differential denoising model capable of protecting an image edge and avoid a step effect occurred during a smoothing process; in the VRDS AI elastic deformation processing, the processing unit 401 is specifically configured to: superimpose positive and negative random distances on an image lattice to form a difference position matrix, and then form a new lattice at a gray level of each difference position, so as to achieve the internal distortion of an image, and then perform rotation, distortion and translation operations on the image.

Embodiments of this application further provide a computer storage medium, wherein the computer storage medium stores a computer program for electronic data exchange, the computer program causes a computer to execute part or all of the steps of any method as recorded in the above method embodiment, and the above computer includes a medical imaging apparatus.

Embodiments of this application further provide a computer program product, the computer program product includes a non-transitory computer-readable storage medium in which a computer program is stored, the above computer program is operable to cause a computer to execute part or all of the steps of any method as recorded in the above method embodiments. The computer program product can be a software installation package, and the above computer includes a medical imaging apparatus.

It should be noted that, for brevity of description, the foregoing method embodiments are described as a series of movement combinations. However, the skilled person in the art should learn that this application is not limited by the movement sequence, because according to this application, some steps can be performed in other order or simultaneously. Second, it also should be known by the skilled person in the art that the embodiments described in the specification are preferred embodiments and the involved actions and modules are not necessarily required for the present application.

In the above embodiments, the descriptions of individual embodiment have their own emphasis, for those parts that are not detailed in one embodiment, please refer to the relevant descriptions of other embodiments.

In the several embodiments provided in this application, it should be understood that the disclosed apparatus may be implemented in other manners. For example, the described apparatus embodiment is merely an example. For example, the above unit division is merely logical function division and may be other division in actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electronic, or other forms.

The above units described as separate parts may or may not be physically separate, and parts displayed as units may or may not be physical units, may be located in one position, or may be distributed on a plurality of network units. It can select some or all of units to achieve the objective of the solution of the present embodiment based on actual requirements.

In addition, functional units in the embodiments of this application may be integrated into one processing unit, or each of the units may exist alone physically, or two or more units are integrated into one unit. The integrated units can be implemented in the form of hardware, and can also be implemented in the form of a software functional unit.

When the above integrated units are implemented in the form of a software functional unit and sold or used as an independent product, the functions may be stored in a computer readable memory. Based on such an understanding, the technical solutions of this application essentially, or the part contributing to the prior art, all or some of the technical solutions may be implemented in a form of a software product. The computer software product is stored in a memory, and includes several instructions for instructing a computer device (which may be a personal computer, a server, or a network device) to perform all or some of the steps of the above methods described in the embodiments of this application. The foregoing memory includes: any medium that can store program code, such as a USB flash disk, a read-only memory (ROM), a random access memory (RAM), a removable hard disk, a magnetic disk, or an optical disc.

The ordinary skill person in the art may understand that all or some of the steps in various methods of the above embodiments can be completed by instructing related hardware through programs, which can be stored in a computer readable memory, which may include: flash disks, Read-Only Memory (ROM), random access memory (RAM) disks or optical disks, etc.

The embodiments of this application are described in detail above, and the principles and implementation way of this application are explained by specific examples. The above embodiments are only used to help understand the method and its core ideas of this application; at the same time, according to the idea of this application, there will be some changes in the specific implementation way and application scope for the ordinary skill person in the art, to sum up, the contents of this specification should not be construed as limitations of this application.

**Claims**

1. A method for AI processing of artery and vein based on Virtual Reality Doctor system (VRDS) 4D medical images, **characterized in that** the method is applied to a medical imaging apparatus; and the method comprises:

determining a bitmap (BMP) data source according to a plurality of scanned images of a target site of a target user;
generating a target medical image data according to the BMP data source; wherein the target medical image data comprises at least a data set of a blood vessel of the target site; the data set of the blood vessel comprises a data set of an artery and a data set of a vein; and first data in the data set of the artery and second data in the data set of the vein are independent of each other; the first data is a data associated with an intersection position of the artery and the vein, the second data is a data associated with the intersection position, the data set of artery is a transfer function result of a cubic space between a surface of the artery and a tissue structure inside the artery, and the data set of the vein is a transfer function result of a cubic space between a surface of the vein and a tissue structure inside the vein; and
performing a 4D medical imaging according to the target medical image data, wherein the intersection position of the artery and the vein presents an overall separation image effect.

2. The method according to claim 1, **characterized in that** the generating the target medical image data according to the BMP data source comprises:

introducing the BMP data source into a preset VRDS medical network model to obtain a first medical image data; wherein the first medical image data comprises a data set of a blood vessel of the target site, the data set of the blood vessel comprises fusion data of the intersection position, data of the artery excluding the intersection position and data of the vein excluding the intersection position; and the data set of the blood vessel is a transfer function result of a cubic space between a surface of the blood vessel and a tissue structure inside the blood vessel;
introducing the first medical image data into a preset cross blood vessel network model and performing spatial segmentation processing on the fusion data at the intersection position by the cross blood vessel network model to obtain the first data and the second data;
synthesizing the first data, the second data, the data of the artery excluding the intersection position and the data of the vein excluding the intersection position to obtain the target medical image data.

3. The method according to claim 1, **characterized in that** the synthesizing the first data, the second data, the data of the artery excluding the intersection position and the data of the vein excluding the intersection position to obtain the target medical image data, comprises:

synthesizing the first data, the second data, the data of the artery excluding the intersection position and the data of the vein excluding the intersection position to obtain a second medical image data;

executing a first preset processing on the second medical image data to obtain the target medical image data, wherein the first preset processing comprises at least one of the following operations: 2D boundary optimization processing, 3D boundary optimization processing and data enhancement processing; and the target medical image data comprises a data set of the target organ, the data set of the artery and the data set of the vein.

4. The method according to claim 3, **characterized in that** the 2D boundary optimization process comprises: acquiring low-resolution information and high-resolution information by sampling multiple times; the 3D boundary optimization processing comprises: 3D convolution, 3D max-pooling and 3D upward convolution layer.

5. The method according to claim 2 or 3, **characterized in that** the introducing the first medical image data into a preset cross blood vessel network model and performing spatial segmentation processing on the fusion data at the intersection position by the cross blood vessel network model to obtain the first data and the second data, comprises:

partitioning the intersection position according to the intersection complexity to obtain a plurality of cross partitions;

screening the fusion data of each cross partition to reduce a data volume of each partition;

introducing the screened data of each partition into the cross blood vessel network model to obtain first partition data and second partition data of each partition;

synthesizing a plurality of pieces of first partition data of the plurality of cross partitions to obtain the first data, and synthesizing a plurality of pieces of second partition data of the plurality of cross partitions to obtain the second data.

6. The method according to claim 5, **characterized in that** the partitioning the intersection position according to the intersection complexity to obtain a plurality of cross partitions, comprises:

acquiring a range of curvature of the intersection position;

obtaining a radius of curvature according to the range of curvature;

performing partitioning according to the radius of curvature to obtain the plurality of cross partitions.

7. The method according to any one of claims 1-6, wherein the performing the 4D medical imaging according to the target medical image data comprises:

screening enhanced data with a quality score greater than a preset score from the target medical image data as VRDS 4D imaging data;

performing 4D medical imaging according to the VRDS 4D imaging data.

8. The method according to any one of claims 1-7, **characterized in that** the determining a BMP data source according to a plurality of scanned images associated with a target organ of a target user comprises:

acquiring a plurality of scanned images collected by a medical device and reflecting internal structural features of human body of the target user;

screening at least one scanned image including the target site from the plurality of scanned images, and taking the at least one scanned image as Digital Imaging and Communications in Medicine (DICOM) data of the target user;

parsing the DICOM data to generate an image source of the target user, wherein the image source comprises Texture 2D/3D image volume data;

executing a second preset processing on the image source to obtain the BMP data source; wherein the second preset processing comprises at least one of the following operations: VRDS limited contrast adaptive histogram equalization, mixed partial differential denoising and VRDS AI elastic deformation processing.

9. The method according to claim 8, **characterized in that** the VRDS limited contrast adaptive histogram equalization comprises the following steps: regional noise ratio limiting and global contrast limiting; dividing the local histogram of the image source into a plurality of partitions; determining a slope of a transform function for each partition according to a slope of a cumulative histogram of a neighborhood of the partition; determining a contrast amplification degree around a pixel value of the partition according to the slope of the transform function; then performing a limit clipping process according to the contrast amplification degree to generate the distribution of effective histograms and a value of a size of an effective available neighborhood; and uniformly distributing these clipped partial histograms to other areas of the histogram;

the mixed partial differential denoising comprises the following steps: enabling a curvature of an image edge to be smaller than a preset curvature through VRDS AI curvature driving and VRDS AI high-order mixed denoising, thereby achieving a mixed partial differential denoising model capable of protecting the image edge and avoiding a step effect occurred during a smoothing process;

the VRDS AI elastic deformation processing comprises the following steps: superimposing positive and negative random distances on an image lattice to form a difference position matrix, and then forming a new lattice at a gray level of each difference position, so as to achieve the internal distortion of an image, and then performing rotation, distortion and translation operations on the image.

10. An apparatus for AI processing of artery and vein based on VRDS 4D medical images, **characterized in that** the apparatus is applied to a medical imaging apparatus; the apparatus for AI processing of artery and vein based on VRDS AI 4D medical image comprises a processing unit and a communication unit, wherein the processing unit is configured to:

determine a bitmap (BMP) data source according to a plurality of scanned images of a target site of a target user;

generate a target medical image data according to the BMP data source; wherein the target medical image data comprises at least a data set of a blood vessel of the target site; the data set of the blood vessel comprises a data set of an artery and a data set of a vein; and first data in the data set of the artery and second data in the data set of the vein are independent of each other, the first data is data associated with the intersection position of the artery and the vein, the second data is data associated with the intersection position, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel; and

perform 4D medical imaging according to the target medical image data through the communication unit, wherein the intersection position of the artery and the vein presents an overall separation image effect.

11. The apparatus according to claim 10, **characterized in that** in an aspect of generating the target medical image data according to the BMP data source, the processing unit is specifically configured to:

introduce the BMP data source into a preset VRDS medical network model to obtain a first medical image data, wherein the first medical image data comprises the data set of the blood vessel of the target site, the data set of the blood vessel comprises fusion data of the intersection position, data of the artery excluding the intersection position and data of the vein excluding the intersection position; and the data set of the blood vessel is a transfer function result of a cubic space between a surface of the blood vessel and a tissue structure inside the blood vessel;

introduce the first medical image data into a preset cross blood vessel network model and perform spatial segmentation processing on the fusion data at the intersection positions by the cross blood vessel network model to obtain the first data and the second data;

synthesize the first data, the second data, the data of the artery excluding the intersection position and the data of the vein excluding the intersection position to obtain the target medical image data.

12. The apparatus according to claim 10, **characterized in that** in an aspect of synthesizing the first data, the second data, the data of the artery excluding the intersection position and the data of the vein excluding the intersection position to obtain the target medical image data, the processing unit is specifically configured to:

synthesize the first data, the second data, the data of the artery excluding the intersection position and the data of the vein excluding the intersection position to obtain a second medical image data;

execute a first preset processing on the second medical image data to obtain the target medical image data, wherein the first preset processing comprises at least one of the following operations: 2D boundary optimization processing, 3D boundary optimization processing and data enhancement processing; and the target medical image data comprises the data set of the target organ, the data set of the artery and the data set of the vein.

13. The apparatus according to claim 12, **characterized in that** the 2D boundary optimization process comprises: acquiring low-resolution information and high-resolution information by sampling multiple times; the 3D boundary optimization processing comprises: 3D convolution, 3D max-pooling and 3D upward convolution layer.

**14.** The apparatus according to claim 11 or 12, **characterized in that** in an aspect of introducing the first medical image data into a preset cross blood vessel network model and performing spatial segmentation processing on the fusion data at the intersection position by the cross blood vessel network model to obtain the first data and the second data, the processing unit is specifically configured to:

partition the intersection position according to the intersection complexity to obtain a plurality of cross partitions;

screen fusion data of each cross partition to reduce a data volume of each partition;

introduce the screened data of each partition into the cross blood vessel network model to obtain first partition data and second partition data of each partition;

synthesize a plurality of pieces of first partition data of the plurality of cross partitions to obtain the first data, and synthesize a plurality of pieces of second partition data of the plurality of cross partitions to obtain the second data.

**15.** The apparatus according to claim 14, **characterized in that** in an aspect of partitioning the intersection position according to the intersection complexity to obtain a plurality of cross partitions, the processing unit is specifically configured to:

acquire a range of curvature of the intersection position;

obtain a radius of curvature according to the range of curvature;

perform partitioning according to the radius of curvature to obtain a plurality of cross partitions.

**16.** The apparatus according to any one of claims 10-15, wherein in an aspect of preforming 4D medical imaging according to the target medical image data, the communication unit is specifically configured to:

screen enhanced data with a quality score greater than a preset score from the target medical image data as VRDS 4D imaging data;

perform 4D medical imaging according to the VRDS 4D imaging data.

**17.** The apparatus according to any one of claims 10-16, **characterized in that** in an aspect of determining a BMP data source according to a plurality of scanned images associated with the target organ of the target user, the processing unit is specifically configured to:

acquire a plurality of scanned images collected by a medical device and reflecting internal structure features of human body of the target user;

screen at least one scanned image including the target organ from the plurality of scanned images; and

take the at least one scanned image as Digital Imaging and Communications in Medicine (DICOM) data of the target user;

parse the DICOM data to generate a image source of the target user, wherein the image source comprises Texture 2D/3D image volume data;

execute a second preset process on the image source to obtain the BMP data source, wherein the second preset process comprises at least one of the following operations: VRDS limited contrast adaptive histogram equalization, mixed partial differential denoising and VRDS AI elastic deformation processing.

**18.** The apparatus according to claim 17, **characterized in that** the VRDS limited contrast adaptive histogram equalization comprises the following steps:

regional noise ratio limiting and global contrast limiting;

dividing the local histogram of the image source into a plurality of partitions;

determining a slope of a transform function for each partition according to a slope of a cumulative histogram of a neighborhood of the partition;

determining a contrast amplification degree around a pixel value of the partition according to the slope of the transform function; then

performing a limit clipping process according to the contrast amplification degree to generate the distribution of effective histograms and a value of a size of an effective available neighborhood; and

uniformly distributing these clipped partial histograms to other areas of the histogram;

the mixed partial differential denoising comprises the following steps:

enabling the curvature of the image edge to be smaller than the preset curvature through VRDS AI curvature driving and VRDS AI high-order mixed denoising, thereby achieving a mixed partial differential denoising model capable of protecting an image edge and avoiding a step effect occurred during a smoothing process;

the VRDS AI elastic deformation processing comprises the following steps: superimposing positive and negative random distances on an image lattice to form a difference position matrix, and then forming a new lattice at a gray level of each difference position, so as to achieve the internal distortion of an image, and then performing rotation, distortion and translation operations on the image.

19. A medical imaging apparatus, **characterized in that** the apparatus comprises a processor, a memory, a communication interface, and one or more programs; the one or more programs are stored in the memory and configured to be executed by the processor, and the programs comprise instructions for executing the steps in the method according to any one of claims 1-9.

20. A computer readable storage medium, **characterized in that** the computer readable storage medium stores a computer program for electronic data exchange, and wherein the computer program causes a computer to execute the method according to any one of claims 1-9.

Fig. 1

| Determining, by medical imaging apparatus, a bitmap (BMP) data source according to a plurality of scanned images of a target site of a target user | S201 |
| Generating , by the medical imaging apparatus, target medical image data according to the BMP data source | S202 |
| Performing , by the medical imaging apparatus, a 4D medical imaging according to the target medical image data | S203 |

Fig. 2

Medical imaging apparatus 300

| Application processor 310 | | Communicati on interface 330 |

Memory 320

One or more programs 321

Fig. 3

400

An apparatus for AI Processing of artery and vein based on VRDS 4D medical images

Storage unit     403

processing unit     401

Communication unit     402

Fig. 4

## INTERNATIONAL SEARCH REPORT

| International application No. |
| --- |
| **PCT/CN2019/101158** |

**A. CLASSIFICATION OF SUBJECT MATTER**

G16H 30/20(2018.01)i

According to International Patent Classification (IPC) or to both national classification and IPC

**B. FIELDS SEARCHED**

Minimum documentation searched (classification system followed by classification symbols)

G16H

Documentation searched other than minimum documentation to the extent that such documents are included in the fields searched

Electronic data base consulted during the international search (name of data base and, where practicable, search terms used)

EPODOC, WPI, CNPAT, CNKI: 血管, 动脉, 静脉, 成像, 成象, 扫描, 医学, 数据, 3D, 4d, blood vessel, artery, vein?, three, four, dimensional, scan, medical, data

**C. DOCUMENTS CONSIDERED TO BE RELEVANT**

| Category* | Citation of document, with indication, where appropriate, of the relevant passages | Relevant to claim No. |
| --- | --- | --- |
| A | CN 109157284 A (GUNAGZHOU DIKA VISION TECHNOLOGY CO., LTD.) 08 January 2019 (2019-01-08) description, paragraphs [0050]-[0078], and figure 1 | 1-20 |
| A | CN 109192308 A (FIRST AFFILIATED HOSPITAL OF XIAN MEDICAL UNIVERSITY) 11 January 2019 (2019-01-11) entire document | 1-20 |
| A | WO 2018222181 A1 (PROXIMIE INC.) 06 December 2018 (2018-12-06) entire document | 1-20 |
| A | CN 109300531 A (SHENZHEN UNIVERSITY) 01 February 2019 (2019-02-01) entire document | 1-20 |
| A | CN 102543044 A (THE SECOND AFFILIATED HOSPITAL OF THIRD MILITARY MEDICAL UNIVERSITY OF THE P.L.A.) 04 July 2012 (2012-07-04) entire document | 1-20 |

☐ Further documents are listed in the continuation of Box C.      ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **16 October 2019** | **28 October 2019** |

| Name and mailing address of the ISA/CN | Authorized officer |
| --- | --- |
| **China National Intellectual Property Administration (ISA/ CN)** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2019/101158**

| Patent document cited in search report | | | Publication date (day/month/year) | Patent family member(s) | | | Publication date (day/month/year) |
|---|---|---|---|---|---|---|---|
| CN | 109157284 | A | 08 January 2019 | None | | | |
| CN | 109192308 | A | 11 January 2019 | None | | | |
| WO | 2018222181 | A1 | 06 December 2018 | US | 2018350073 | A1 | 06 December 2018 |
| CN | 109300531 | A | 01 February 2019 | None | | | |
| CN | 102543044 | A | 04 July 2012 | None | | | |

Form PCT/ISA/210 (patent family annex) (January 2015)